# EUROPEAN PATENT SPECIFICATION

(11) **EP 0 868 903 B1**
(45) Date of publication and mention of the grant of the patent: **08.11.2006**
(21) Application number: 97122384.7
(22) Date of filing: 18.12.1997
(51) Int. Cl.: A61Q 11/00, A61K 8/90

(54) **Liquid polyoxyalkylene compounds in oral care compositions and dentifrice formulations**
Flüssige Polyoxyalkylenverbindungen in Mundpflegemittel und Zahnpasta
Composés polyoxalkylènes liquides dans des compositions d'hygiene bucco-dentaire et dentifrices

(30) Priority: 30.12.1996 US 774663; 31.12.1996 US 775328
(43) Date of publication of application: 07.10.1998
(73) Proprietor: BASF CORPORATION, Mount Olive, New Jersey 07828-1234 (US)
(72) Inventor: Gopalkrishnan, Sridhar, Woodhaven, MI 48183 (US); Holland, Richard, Flanders, NJ 07836 (US); Dailey, James S., Grosse Ile, MI (US); Otten, Jay Gregory, Flat Rock, MI 48134 (US); Amarasekara, Jay, Clifton Park, NY 12065 (US)
(74) Representative: Abel, Manfred

(56) References cited:
- EP-A- 0 604 158
- EP-A- 0 850 972
- US-A- 3 976 765
- US-A- 4 476 107
- US-A- 5 096 698
- US-A- 5 187 191
- US-A- 5 374 368
- PATENT ABSTRACTS OF JAPAN vol. 1995, no. 08, 29 September 1995 (1995-09-29) & JP 07 126690 A (SANYO CHEM IND LTD), 16 May 1995 (1995-05-16)

## Description

The present invention relates to an oral care formulation comprising a liquid, polyalkylene glycol carrier and a liquid polyoxyalkylene compound miscible in said liquid, polyalkylene glycol carrier.

The present invention also relates to oral care compositions comprising liquid polyoxyalkylene compounds (nonionic surfactants) suitable as additives with multifunctional benefits for aqueous oral care compositions. These copolymers exhibit utility as gelling agents for aqueous toothpaste formulations, as foam boosting additives for oral care formulations, and as solubilizers in mouthwash formulations.

Polyoxyalkylene block copolymers are well known to those skilled in the non-ionic surfactant art. Specifically, US 3,740,421 (Schmolka) assigned to BASF discloses aqueous gels prepared using a block copolymer of polyoxyethylene/polyoxypropylene suitable for pharmaceutical and personal care compositions. US 3,639,574 (Schmolka) assigned to BASF discloses polyoxyethylene/polyoxypropylene block copolymers as gelling agents for hydrogen peroxide compositions. US 4,465,663 (Schmolka) assigned to BASF discloses polyoxybutylene/polyoxyethylene block copolymers as gelling agents for aqueous gels useful in personal care and pharmaceutical applications. These compounds are sold by the BASF Corporation under the PLURONIC® tradename.

Additionally, US 4,272,394 and US 4,411,810 disclose the use of polyoxyalkylene block copolymers in machine dishwashing applications. US 4,925,988 discloses a nonionic surfactant employing a specific combination of alkanol, ethylene oxide and propylene oxide useful in an automatic dishwashing application.
U.S. 5,374,368 describes the use of liquid EO/PO/EO triblock copolymers (PLURONIC® L 31 and L 35 surfactants) in stable hydrogen peroxide releasing dental care compositions at levels of 55 - 90% by weight of the dental care composition. U.S. 3,740,421 discloses gel forming solid EO/PO/EO triblock copolymers useful in cosmetic and personal care formulations at levels of approximately 20 - 25% by weight. Preferred solid EO/PO/EO triblock copolymers have a molecular weight of 4,600 - 16,000. Said solid EO/PO/EO triblock copolymers form a gel when added to an aqueous solution. U.S. 3,867,533 discloses aqueous gel compositions containing solid EO/PO/EO triblock copolymers, having a molecular weight of 6,450 - 20,000 useful at levels of approximately 20% by weight. Said compositions are useful in preparing cosmetic formulations. U.S. 4,465,663 discloses clear aqueous cosmetic gels containing solid EO/BO(butylene oxide)/EO triblock copolymers at levels of approximately 20%. U.S. 5,035,880 discloses a stable dentifrice compositions containing a cetylpyridinium bactericide and EO/PO/EO solid triblock copolymers (PLURONIC® F 127 surfactant), and polyethylene glycol at levels of 15 - 80% by weight. U.S. 4,476,107 discloses a mouthwash containing EO/BO(butylene oxide)/EO triblock copolymers at levels of 0.5 - 5.0% by weight. U.S. 5,057,307 discloses oral hygiene gels containing non-ionic surfactants, coating substances; and viscosifiers. Said non-ionic surfactants are the PLURONIC® F 108 and F 127 surfactants available from BASF Corporation, Mt. Olive, New Jersey. U.S. 5,256,396 discloses a topical composition comprising an EO/PO/EO solid triblock copolymer (PLURONIC® F 127 surfactant) used at a level of more than 10% to about 17% by weight.
EPO-546-627A discloses mouthwash compositions comprising solid EO/PO/EO triblock copolymers such as PLURONIC® L 108, F88 surfactants at levels of 0.5 - 3% by weight. U.S. 5,073,368 discloses mouthwashes containing solid EO/PO/EO triblock copolymers such as PLURONIC® F 87 surfactant at levels of 0.1 - 3% by weight. WO 93/13750 discloses an ocular cleansing composition comprising solid PLURONIC® F 87 and paste PLURONIC® P 85 EO/PO/EO triblock copolymers. PLURONIC® P 85 surfactant is 4 - 9% by weight of the cleansing composition, PLURONIC® F 87 surfactant is 0.5 - 2% by weight of the cleansing composition. Finally, U.S. 5,096,698 discloses a dental creme composition containing a non-ionic triblock liquid EO/PO/EO copolymer or a solid triblock EO/PO/EO copolymer at levels of 0.1 - 5% by weight. Said copolymers help to prevent phase separation. PLURONIC® F 108 surfactant (solid) is most preferred, followed by PLURONIC® F 87, PLURONIC® F 127, and PLURONIC® L 72 surfactants. US 4,272,394 discloses novel, low-foaming nonionic surfactant for machine dishwashing compositions. US 4, 411,810 discloses a low foaming, low cloud point, nonionic surfactant for machine dishwashing compositions. JP 47-48366 B4 discloses a process for producing tasteless, liquid, heteric polyoxyalkylene compounds of molecular weight 1000 or higher. US 5,187, 191 discloses polyoxyalkylene block copolymers in agricultural formulations. US 5,496,542, US 5,374,368, and US 5,424,060 disclose the use of polyoxyalkylene compound for formulating a stable percarbonate formulation as well as a dentifrice composition.

The present invention relates to oral care compositions, such as dentifrice formulations. Dentifrice formulations typically contain substantial amounts of humectants. Humectants help the formulation retain its moisture, thus, preventing the formulation from hardening when the container cap is left open for extended periods. Typical humectants employed in such formulations are glycerol, sorbitol which are usually the preferred humectants because of their sweet taste. Other humectants which are also used are polyethylene glycols of low molecular weight typically between 200 - 600. Liquid polyethylene glycols constitute an excellent choice as a carrier for formulating a non-aqueous, dentifrice formulation since they come with several advantages such as good hygroscopicity, low viscosity, good compatibility with several dentifrice ingredients, low volatility, low cost, low toxicity, low odor and low pour point. More recently, toothpaste compositions are being formulated without any significant incorporation of water. Examples of such toothpaste compositions are those that cannot tolerate the presence of significant levels of water due to concerns related to decomposition of key ingredients leading to loss of activity, or reactivity of ingredients, for example, baking soda and a peroxygen compound such as, hydrogen peroxide or sodium percarbonate. The formulation and increased stability of such toothpaste compositions is achieved by employing a non-aqueous carrier typically selected from low molecular weight liquid polyethylene glycols. Dentifrice formulations also contain an anionic surfactant, typically selected from sulfate esters of C_{10 - 18} alcohols. An example of such a anionic surfactant is sodium lauryl sulfate. The primary function of the anionic surfactant is to provide efficient foaming action during brushing. However, in many instances the foaming action provided by the anionic surfactant alone is inadequate and often additional ingredients have to be added in combination with the anionic surfactant to achieve the desired foam profile during brushing. A common ingredient that is typically selected to achieve additional foam boosting are the high molecular weight, solid block copolymers of ethylene oxide and propylene oxide. Examples of such solid block copolymers are those that have a number average molecular weight over 8000 and also contain at least 50% ethylene oxide. Particularly preferred are those that contain between 70% - 80% ethylene oxide and have a number average molecular weight greater than 9000.

Further, a limitation also exists with the use of the high molecular weight, solid, block copolymers of ethylene oxide and propylene oxide in a non-aqueous dentifrice formulation because they are insoluble in the liquid polyalkylene glycol carrier. Dissolution of the solid block copolymer in the liquid polyalkylene glycol carrier is achieved by heating the two components until a single clear phase is achieved. Unfortunately, upon cooling back to ambient temperatures, the solid block copolymer has a tendency to phase separate leading to the formation of a heterogenous mixture. The formation of a heterogenous phase is particularly problematic since it may lead to a preferential partitioning of certain dentifrice components in any one phase and an altering of the homogeneity of the formulation. This altering of the homogeneity of the formulation is particularly noticeable when the dentifrice formulation undergoes multiple heating and cooling cycles during transportation and storage.

The Applicants have solved these aforementioned problems in the art. Surprisingly, Applicants have discovered that if the solid block copolymers are rendered liquid during their preparation, by addition of small amounts of a C₃ or higher alkylene oxide to the crystalline, hydrophilic portion of the solid block copolymer to produce a liquid polyoxyalkylene compound of similar hydrophilicity and molecular weight, then a single, clear, homogenous phase is achieved upon adding to the liquid, polyoxyalkylene glycol carrier. An additional advantage with the use of the high molecular weight liquid polyoxyalkylene compounds of this invention is that upon addition to the polyalkylene glycol carrier, they are readily miscible and form a clear, homogenous, single phase composition, and do not have to be heated to achieve dissolution.

Polyoxyalkylene block copolymers have been used as non ionic surface active agents for many years. In general they have both hydrophobic and hydrophilic blocks which in combination give these compounds their surfactant properties. These products have been described before in numerous references. For example, Lunsted was the first to describe them in U.S. 2,674,619. Heterization of both the hydrophobic and hydrophilic blocks of non-ionic block copolymer surfactants is well known in the art of surfactant chemistry. Lunsted, et.al. in U.S. 3,022,335, discloses surface active polyoxyalkylene compounds having plurality of heteric polyoxypropylene-polyoxyethylene chains, more specifically a heteric oxyalkylene-oxyethylene chain or chains as a hydrophobic nucleus to which oxyethylene chains were attached as the hydrophilic block. Patton, et. al., in U.S. 3,101,374, discloses surface active mixtures of polyoxyalkylene compounds composed of hydrophobic oxyalkylene chains or blocks which are condensed with a plurality of heteric oxyethylene-oxyalkylene chains. Paton also discloses that the resultant products were liquefied by addition of alkylene oxide, in a large enough percentage, to the heteric hydrophilic block. Otten & Schoene in U.S. 5,187,191, disclose an aqueous pesticide dispersion composed of a water insoluble pesticide, a liquid polyoxyalkylene compound having a plurality of heteric polyoxypropylene polyoxyethylene chains and water.

The aqueous gel properties and uses of solid polyalkylene/polyoxyethylene block polymers are well documented. US 3,639,574 (Schmolka) assigned to BASF discloses polyoxyethylene/polyoxypropylene block copolymers as gelling agents for hydrogen peroxide compositions. In Lutz, U.S. 4,011,309, dentifrice compositions are disclosed which are composed of aqueous gel formulations that incorporate polyoxyethylene-polyoxypropylene block copolymers as the main gel component. Schmolka, in U.S. 4,343,785, discloses an aqueous gel dentifrice comprising polyoxybutylenepolyoxyethylene block copolymers. Schmolka also discloses mouthwash compositions which utilize polyoxybutylene-polyoxypropylene block copolymers as solubilizers.

Further, US 3,740,421 (Schmolka) assigned to BASF discloses aqueous gels prepared using a triblock copolymer of polyoxyethylene/polyoxypropylene/polyoxyethylene suitable for pharmaceutical and personal care compositions at approximately 20 - 25% by weight. US 4,465,663 (Schmolka) assigned to BASF discloses polyoxybutylene/polyoxyethylene block copolymers as gelling agents for aqueous gels useful in personal care and pharmaceutical applications. Some of these compounds are sold by the BASF Corporation under the PLURONIC® tradename.

Additionally, US 4,272,394 and US 4,411,810 disclose the use of polyoxyalkylene block copolymers in machine dishwashing applications. US 4,925,988 discloses a nonionic surfactant employing a specific combination of alkanol, ethylene oxide and propylene oxide useful in an automatic dishwashing application.
U.S. 5,374,368 describes the use of liquid EO/PO/EO triblock copolymers (PLURONIC® L 31 and L 35 surfactants) in stable hydrogen peroxide releasing dental care compositions at levels of 55-90% by weight of the dental care composition. U.S. 3,867,533 discloses aqueous gel compositions containing solid EO/PO/EO triblock copolymers, having a molecular weight of 6,450 - 20,000 useful at levels of approximately 20% by weight. Said compositions are useful in preparing cosmetic formulations.
U.S. 5,035,880 discloses a stable dentifrice compositions containing a cetylpyridinium bactericide and EO/PO/EO solid triblock copolymers (PLURONIC® F 127 surfactant), and polyethylene glycol at levels of 15 - 80% by weight. U.S. 4,476,107 discloses a mouthwash containing EO/BO (butylene oxide)/EO triblock copolymers at levels of 0.5 - 5.0% by weight. U.S. 5,057,307 discloses oral hygiene gels containing non-ionic surfactants coating substances; and viscosifiers. Said non-ionic surfactants are PLURONIC® F 108 and F 127 surfactants available from BASF Corporation, Mt. Olive, New Jersey. U.S. 5,256,396 discloses a topical composition comprising an EO/PO/EO solid triblock copolymer (PLURONIC® F 127 surfactant) used at a level of more than 10% to about 17% by weight. EPO-546-627A discloses mouthwash compositions comprising solid EO/PO/EO triblock copolymers such as PLURONIC® L 108, F 88 surfactants at levels of 0.5 - 3% by weight. U.S. 5,073,368 discloses mouthwashes containing solid EO/PO/EO triblock copolymers such as PLURONIC® F 87 surfactant at levels of 0.1 - 3% by weight. WO 93/13750 discloses an ocular cleansing composition comprising solid PLURONIC® F 87 and paste PLURONIC® P 85 EO/PO/EO triblock copolymers. PLURONIC® P 85 surfactant is 4 - 9% by weight of the cleansing composition, PLURONIC® F 87 surfactant is 0.5 - 2% by weight of the cleansing composition. U.S. 5.096,698 discloses a dental creme composition containing a non-ionic triblock liquid EO/PO/EO copolymer or a solid triblock EO/PO/EO copolymer at levels of 0.1-5% by weight. Said copolymers help to prevent phase separation. PLURONIC® F 108 surfactant (solid) is most preferred, followed by PLURONIC® F 87, PLURONIC® F 127, and PLURONIC® L 72 surfactants. US 4,272,394 discloses novel, low-foaming nonionic surfactant for machine dishwashing compositions. US 4,411,810 discloses a low foaming, low cloud point, nonionic surfactant for machine dishwashing compositions. Finally, JP 47-48366 B4 discloses a process for producing tasteless, liquid, heteric polyoxyalkylene compounds of molecular weight 1000 or higher. US 5,187,191 discloses polyoxyalkylene block copolymers in agricultural formulations. US 5,496,542, US 5,374,368, and US 5,424,060 disclose the use of a polyoxyalkylene compound for formulating a stable percarbonate formulation as well as a dentifrice composition.

Clearly, it is known to those ordinarily skilled in the art that solid block copolymers of ethylene oxide and propylene oxide are useful in aqueous dentifrice compositions. However, a typical disadvantage with the solid blockcopolymers is their physical form. Triblock copolymers of ethylene oxide and propylene oxide, wherein the ethylene oxide content in the copolymer is over 70%, are typically solids above a MW of 3000. Solid, triblock copolymer of ethylene oxide and propylene oxide can pose handling problems during the manufacture of aqueous dentifrice compositions. Such difficulties typically arise due to their high melting points. In many cases, the solid triblock copolymers have to be converted to their molten state during processing.

The Applicants have surprisingly discovered that when the solid triblock copolymers are rendered liquid via a carefully controlled addition of a heteric mixture of ethylene oxide and propylene oxide to the hydrophobic propyleneoxide block, the resulting product essentially retains the properties of the solid block copolymer and has the benefit of ease of handling in formulating personal care formulations. Specifically, the Applicants' invention overcomes the problems in the art by preparing a liquid copolymer useful in aqueous oral care compositions while essentially retaining the properties of the solid block copolymer. Thus the liquid copolymer of the invention affords ready solubility in aqueous oral care compositing or solubilizing agents for such compositions.

Additionally, the references listed hereinabove describe methods of preparation for many different polyoxyalkylene polyether surfactants. However, an undesirably broad distribution of molecular weights is obtained both in the hydrophobe and hydrophile portion of the polymer. The broad distribution of molecular weight products, which result from the anionic living polymerization, may be narrowed to some extent by reduction of a side reaction which develops during propylene oxide additions. Some of the side reactions may result in terminal unsaturation. Cuscurida in U.S. 3,393,243, discloses a method for preparation of propylene oxide based polyether polyols with reduced content of terminal unsaturation by using CsOH as a catalyst. Ott in U.S. 4,764,567, discloses a method of preparation for narrow molecular weight distribution products by using CsOH during the ethylene oxide addition. Finally, Otten in U.S. 4,902,834, discloses an improved process for the preparation of capped polyoxyalkylene block polyethers using CsOH.

Further, the Applicants have also discovered that the use of cesium hydroxide as a catalyst in the synthesis of the liquid polyoxyalkylene block copolymers of the present invention resulted in further improvement in the gel characteristics of the liquid polymer relative to a potassium hydroxide catalyzed material.

The present invention relates to an oral care composition comprising:
(a) a liquid polyalkylene glycol carrier and;
(b) a liquid polyoxyalkylene compound miscible in said liquid polyalkylene glycol carrier, wherein said:
   liquid polyalkylene glycol carrier (a) is selected from:
      (i) liquid polyethylene glycols having a molecular weight of no more than 600; or
      (ii) liquid polyoxyalkylene glycols having a molecular weight greater than 600 and less than 3000 of Formula I or mixtures of (i) and (ii):

         I-[(EO)ₐ(AO)_{b}-M]_{y}; Formula I

         wherein in Formula I:
         I is an initiator or a mixture of initiators having at least two carbon atoms and at least two or more hydroxyl substituents;
         AO is a C₃₋₄ alkylene oxide or a mixture of C₃₋₄ alkylene oxides;
         EO is ethylene oxide;
         wherein, further EO and AO can be distributed randomly and/or arranged in a block sequence;
         M is hydrogen or an alkali metal or alkaline earth metal;
         a is an integer from 6 - 22;
         b is an integer from 1 - 9;
         y is an integer from 2 - 4;
and further provided that
said liquid polyoxyalkylene compound (b), which is miscible in (a), is represented by Formula IIa:

I[(AO)ₐ-(XO)-M]ₓ Formula IIa

wherein in Formula IIa:
I is an initiator or a mixture of initiators having at least two carbon atoms and two hydroxyl substituents;
AO is propylene oxide;
XO is (EO)_{b}(AO)_{c} wherein EO is ethylene oxide and EO and AO are distributed randomly;
M is hydrogen or an alkali metal or an alkaline earth metal;
a is an integer from 15 - 35;
b is an integer from 50 - 150;
c is an integer from 7 - 90;
x is 2.

The molecular weight range for Formula I is from 600 - 3000.
The more preferred values for molecular weight are from 1000 - 2500.
The most preferred molecular weight is from 1500 - 2000.
The molecular weight of the liquid polyoxyalkylene compound of Formula IIa is from 8000 - 28,000, more preferred is from 9000 - 24,000, and most preferred is from 10,000 - 19,500.

In Formula I, a is an integer from 6 - 22, preferably, from about 7 - 20 and more preferably from 10 - 16; b is an integer from 1 - 9, preferably from 1 -7 and more preferably from 2 - 5; y is an integer from 2 - 4, preferably from 2 - 3 and more preferably 2.

In Formula IIa, a is an integer from 15 - 35, preferably 20 - 32, more preferably 24 - 31; b is an integer from 50 - 150, preferably 65 - 135; more preferably 70 - 120; c is an integer from 7 - 90, preferably 10 - 70; more preferably 15 - 45; x is 2.

The initiator (I) in Formula I is preferably selected from the group consisting of propylene glycol, dipropylene glycol, ethylene glycol, and diethylene glycol, and glycerol. Most preferably the initiator is propylene glycol.

The initiator (I) in Formula IIa is preferably selected from the group consisting of propylene glycol, dipropylene glycol, ethylene glycol, and diethylene glycol. Most preferably the initiator is propylene glycol.

In Formula I and IIa, EO is ethylene oxide.

In Formula I, AO is an alkylene oxide moiety having 3 - 4 carbons, preferably, said alkylene oxide moiety is propylene oxide or butylene oxide, most preferably propylene oxide. In Formula IIa, AO is propylene oxide.
Further, EO and AO in Formula I can be distributed randomly and/or arranged in a block sequence. In Formula IIa, the distribution must be random.

In Formula I and IIa, M is H or a cation selected from the group including, but not limited to, lithium, calcium, potassium, and cesium, most preferably potassium or hydrogen.

### Description of the Method of Preparation of the Liquid Polyalkylene Glycol Carrier and Liquid Polyoxyalkylene Compound Miscible in Said Liquid Polyalkylene Glycol Carrier of the Present Invention

### Preparation of the Liquid Polyoxyalkylene Compound of the Invention

The liquid polyoxyalkylene compound of the invention was prepared by conventional techniques in 1 or 2 gallon stainless steel autoclaves that were equipped with stirring, pressure gauge, thermocouple and addition tube. For instance, an initiator, in all cases propylene glycol, and catalyst were vacuum stripped in a stainless steel stirred autoclave at 120°C to remove water. The formation of a alkylene oxide block was achieved by the addition of propylene oxide at 105°C under a nitrogen atmosphere with addition rates set such that the overall pressure does not exceed 90 psig. To the polyoxypropylene block was added a mixture of ethylene oxide and propylene oxide to form a mixed oxide hydrophilic block. Mixed oxide additions were carried out at 135°C with the same pressure constraints as above. The final products were neutralized by addition of 85% phosphoric acid and inhibited by addition of 100 ppm t-butylhydroxytoluene.

### Preparation of the Liquid Polyalkylene Glycol Carrier of the Invention

A two gallon stainless steel autoclave was charged with 1089 g of propylene glycol, 46.6 g of 45% potassium hydroxide and purged with nitrogen. The contents were heated to 80°C and stripped to remove volatiles for 2 hours. The contents were heated to 130°C and 4412.9 g of ethylene oxide added over a period of six hours. After the addition was finished the mixture was kept at 130°C for one hour and then volatiles stripped for 1/2 hour.

A five gallon stainless steel autoclave was charged with 3075.6 g of the above material and purged with nitrogen. The material was heated to 116°C and 11,168 g of a mixture of 76.8% ethylene oxide and 23.2% propylene oxide added over a period of 9.5 hours. After the addition was complete, the mixture was kept at 116°C for an additional two hours. A final charge of 1762 g of ethylene oxide was added over two hours and then kept at 116°C for 2.5 hours. The mixture was stripped for 1/2 hour, cooled to 80°C and 30.1 g of 50% hypophosphorous acid added. The mixture was agitated for 1/2 hour in the autoclave and then discharged.

### The Utility of the Present Invention

In order to illustrate the utility of the present invention, _{A}p-plicants prepared binary blends comprising a liquid polyalkylene glycol and a high molecular weight, solid block copolymer with a molecular weight greater than 8000. Said binary blends were then compared to blends of the present invention comprising Compound A of Formula II (liquid) and Compound B of Formula I (liquid). See Table-1.

The binary (solid /liquid) blends were prepared by first mixing the solid block copolymer with the liquid polyalkylene glycol and then heating the blend above the melting point of the solid block copolymer to obtain a clear, single phase composition. The resulting clear composition was then gradually allowed to cool to ambient temperatures while being continually stirred at 100 rpm on a LIGHTNIN mixer. The final composition was then stored at ambient temperatures and physical stability of the composition was noted.

The binary (liquid/liquid) blends of the present invention were prepared by simply mixing the components (a) liquid polyalkylene glycol carrier, and (b) liquid polyalkylene compound as defined hereinabove.

As the Table 1 indicates, all blends comprising a liquid, polyethylene glycol of molecular weight 400 and the solid, block copolymer were unstable on storage and separated into two phases. This is particularly evident when the samples are stored at 45⁰C. Clearly, Test 8 and 9 (the present invention) illustrates the utility of the present invention in maintaining the stability and uniformity of dentifrice formulations.

However, when the solid block copolymer was replaced with the liquid polyoxyalkylene compound of Formula IIa (Compound A), then solubility was instantaneous and the resulting composition was a single phase, clear, stable, homogenous liquid. The table further shows that when the liquid, polyethylene glycol of molecular weight 400 is replaced with the liquid polyalkylene glycol of Formula I (Compound B), a clear, single phase, stable, homogenous composition is achieved upon blending with the liquid polyoxyalkylene compound of Formula IIa (Compound A).

**Table 1**

| Test # | Component A | Component B | Remarks | Stability |
|---|---|---|---|---|
| 1 | PEG 400 | Pluronic F38 | Heat to dissolve | Two phases - Unstable |
| 2 | PEG 400 | Pluronic F68 | Heat to dissolve | Two phases - Unstable |
| 3 | PEG 400 | Pluronic F87 | Heat to dissolve | Two phases - Unstable |
| 4 | PEG 400 | Pluronic F88 | Heat to dissolve | Two phases - Unstable |
| 5 | PEG 400 | Pluronic F98 | Heat to dissolve | Two phases - Unstable |
| 6 | PEG 400 | Pluronic F108 | Heat to dissolve | Two phases - Unstable |
| 7 | PEG 400 | Pluronic F127 | Heat to dissolve | Two phases - Unstable |
| 8 | PEG 400 | Compound A of Formula IIa | Readily dissolves | Clear, single phase - Stable |
| 9 | Compound B of Formula I | Compound A of Formula IIa | Readily dissolves | Clear, single phase - Stable |

In each blend, the weight ratio of the liquid polyalkylene glycol to the solid block copolymer or Component B was 90:10.

### Explanation of terms in Table 1

PEG400 - Liquid polyethylene glycol of molecular weight 400. Available from BASF Corporation as PLURACOL E-400 polyethylene glycol.

PLURONIC^{®} is a registered trademark of BASF. Pluronic surfactant is a triblock copolymer of ethylene oxide and propylene oxide available from BASF. Pluronic F surfactant refers to the solid, triblock copolymers of ethylene oxide and propylene oxide.

PLURONIC^{®} F38 surfactant is a solid, triblock copolymer of ethylene oxide and propylene oxide with a average molecular weight of 4700.

PLURONIC^{®} F68 surfactant is a solid triblock copolymer of ethylene oxide and propylene oxide with a average molecular weight of 8400.

PLURONIC^{®} F87 is a solid triblock copolymer of ethylene oxide and propylene oxide with a average molecular weight of 7700.

PLURONIC^{®} F88 is a solid triblock copolymer of ethylene oxide and propylene oxide with a average molecular weight of 11400.

PLURONIC^{®} F98 is a solid triblock copolymer of ethylene oxide and propylene oxide with a average molecular weight of 13000.

PLURONIC^{®} F108 is a solid triblock copolymer of ethylene oxide and propylene oxide with a average molecular weight of 14600.

PLURONIC^{®} F127 is a solid triblock copolymer of ethylene oxide and propylene oxide with a average molecular weight of 12600.

Compound A of Formula IIa, wherein I is propylene glycol, AO is propylene oxide, and a is 29, b is 78, c is 15, and x is 2, and M is hydrogen.

Compound B of Formula I, wherein I is propylene glycol, AO is propylene oxide, and a is 18, b is 3, and M is hydrogen.

### Preparation of Oral Care (Dentifrice) Compositions Containing the Liquid Polyalkylene Glycol Carrier and Liquid Polyoxyalkylene Compound Miscible in Said Liquid Polyalkylene Glycol Carrier of the Present Invention

The liquid polyalkylene glycol carrier and liquid polyoxyalkylene compound miscible in said liquid polyalkylene glycol carrier of the present invention are present in oral care compositions such as dentifrice compositions at a preferred level of 1 - 99%, more preferably at a level of 20 - 79%; most preferably at a level of 30 - 50% by weight of the oral care composition. Generally, the level of incorporation depends on the end use of the liquid polyalkylene compound of the invention. If they function as carriers, for example, in a essentially non-aqueous dentifrice formulation, then higher use levels are required in the formulation.

Oral care such as dentifrice formulations also contain other ingredients such as surfactants selected from anionic surfactants which include sodium lauryl sulphate; sodium alkyl glyceryl ether sulfonate; alkyl benzene sulfonates. Other anionic surfactants also include oxyalkylates of C₆ - C₁₈ alcohols. It is also known to those skilled in the art to use solid block copolymers of polyoxyethylene and polyoxypropylene to further provide a boost in the foaming performance of the dentifrice composition. Such solid block copolymers of ethylene oxide and propylene oxide are excluded from the scope of this invention. Further, small amounts of cationic surfactants, having a quaternary nitrogen, which show compatibility with the nonionic carrier blends of this invention can also be used. Various other materials may also be used in the formulating of personal care products. For example, peroxygen compounds such as hydrogen peroxide, sodium percarbonate, can be used in such dentifrice compositions. Dental abrasives consisting of finely divided silica, or calcium carbonate, sodium bicarbonate, calcium pyrophosphate, and hydrated alumina are added for polishing performance. Additionally, thickening agents such as collodial silica, xanthan gum, gum arabic, hydroxyethylcellulose, polyvinylpyrrolidone, gum tragacanth, carragennan can also be used to provide sufficient thickening consistency to the formulation. Also, flavoring agents such as peppermint, spearmint oils or preservatives, opacifying agents, buffer salts, sweeteners, anti-bacterial agents anti-calculus agents or antiplaque agents, anti-inflammatory agents, anti-caries agents such as the fluoride salts can also be included in small amounts. Polymeric agents which accelerate the transport of active materials can also be included. Additionally, dentifrice compositions may contain small amounts of water.

Personal care products, such as dentifrices, are formulated according to methods known to those skilled in the art. Representative personal care product formulations are disclosed in: Cosmetics, Science and Technology, 2nd Edition, Vol. 1, Edited by M.S. Balsam, et al., and A Formulary of Cosmetic Preparations, Michael and Irene Ash, Chemical Publishing, N.Y., N.Y., both incorporated by reference herein.

The following dentifrice composition serves to illustrate the utility of the present invention. All percentages are weight percent (%) of the total composition unless otherwise indicated.

### Dentifrice Composition:

- 1 - 55%: abrasive, selected from anhydrous dicalcium phosphate, calcium carbonate, calcium pyrophosphate and sodium bicarbonate;
- 0 - 0.6%: of a fluoridating agent, including stannous fluoride, sodium fluoride and sodium monofluorophosphate;
- 2-10%: binders, including gum karaya, tragacanth USP, sodium alginate, Irish moss and methyl cellulose;
- 0 - 5%: thickening agent, including collodial silica;
- 0 - 10%: of a peroxygen source, including, hydrogen peroxide and sodium percarbonate;
- 0 - 8%: surfactants, including sodium lauryl sulfate, sodium-N-lauryl sarcosinate and dioctyl sodium sulfosuccinate;
- 0.1 - 10%: of the liquid polyoxyalkylene compound (b) represented by Formula IIa:

I[(AO)ₐ-(XO)-M]ₓ Formula IIa

wherein in Formula IIa:
I is an initiator or a mixture of initiators having at least two carbon atoms and two hydroxyl substituents;
AO is propylene oxide;
XO is (EO)_{b}(AO)_{c} wherein EO is ethylene oxide and EO and AO are distributed randomly;
M is hydrogen or an alkali metal or an alkaline earth metal;
a is an integer from 15 - 35;
b is an integer from 50 - 150;
c is an integer from 7 - 90;
x is 2;
- 5 - 70%: carriers selected from the group glycerin; propylene glycol, sorbitol, liquid polyethylene glycol, and the liquid polyalkylene glycols (a) represented by Formula I:

I-[(EO)ₐ(AO)_{b}-M]_{y}; Formula I

wherein in Formula I:
I is an initiator or a mixture of initiators having at least two carbon atoms and at least two or more hydroxyl substituents;
AO is a C₃₋₄ alkylene oxide or a mixture of C₃₋₄ alkylene oxides;
EO is ethylene oxide;
wherein, further EO and AO can be distributed randomly and/or arranged in a block sequence.
M is hydrogen or an alkali metal or alkaline earth metal;
a is an integer from 6 - 22;
b is an integer from 1 - 9;
y is an integer from 2 - 4.

The present invention also relates to an oral care composition comprising 0.1 - 50% of the liquid polyoxyalkylene compound having the formula II:

I'((AO)ₐ-(XO)-M]ₙ Formula II

wherein I' is an initiator or a mixture of initiators having at least two carbon atoms wherein at least one carbon atom must have at least one substituent selected from -OH, or -NH₂;
AO is a C₃₋₄-alkylene oxide or mixtures of C₃₋₄ alkylene oxide arranged in a block sequence;
XO is (EO)_{b}(AO)_{c} wherein EO is ethylene oxide and EO and AO are distributed randomly;
M is hydrogen or alkali metal or alkaline earth metal;
a is an integer from 15 - 35;
b is an integer from 50 - 150;
c is an integer from 7 - 90;
n is an integer from 1-3.

The liquid polyoxyalkylene compounds of the formula IIa form a sub-group to the compounds of the formula II.

Preferred MWs for the compounds of the formula II are from 8000 - 28000, more preferred 9000 - 24000, most preferred 10500 - 19500.

In the formula II, a is an integer from 15 - 35, more preferably 20 - 32, most preferably 24 - 31,
b is an integer from 50 - 150, more preferably 65 - 135, most preferably 70 - 120,
c is an integer from 7 - 90, more preferably 10 - 70, most preferably 15 - 45,
n is an integer from 1 - 3, more preferably 2 - 3, most preferably 2.

The initiator is preferably selected from the group consisting of propylene glycol, dipropylene glycol, ethylene glycol, and diethylene glycol, and glycerol, most preferably the initiator is propylene glycol.

Triblock copolymers of EO and PO (EO/PO/EO) are excluded from the scope of this invention.

### The Preparation of the Polyoxyalkylene Copolymers of the Present Invention

All polyoxyalkylene block copolymers of the present invention were prepared by conventional techniques in 1 or 2 gallon stainless steel autoclaves that were equipped with stirring, pressure gauge, thermocouple and addition tube. For instance, an initiator₁ in this case propylene glycol, and catalyst, selected from the group including but not limited to NaOH, KOH, CsOH, were vacuum stripped in a stainless steel stirred autoclave at 120°C to remove water formation of the polyoxypropylene block was achieved by the addition of propylene oxide at 105°C under a nitrogen atmosphere with addition rates set such that the overall pressure does not exceed 90 psig and the residual PO concentration at the end of addition does not exceed 18%. To the polyoxypropylene block was added a mixture of ethylene oxide and propylene oxide to form a mixed oxide hydrophilic block. Mixed oxide additions were carried out at 1 35⁰C with the same pressure constraints as above. Other products produced with polyoxyethylene hydrophilic blocks used an ethylene oxide addition temperatures of 135°C. The catalyst content for these syntheses was between 0.06% - 0.50% final catalyst level. The ethylene oxide and mixed oxide charges were added under a 34 psig nitrogen pad with addition rates to keep the vapor phase EO concentration below the safe explosive limit which can be calculated according to Siwek in Siwek, R., Rozenberg, E., ^{.'} Ethylyene Oxide Vapor Decomposition - Process and Protective Measures" Zeitschrift fur die Fett-, Öl-, Tensid-. Kosmetik und Pharmaindustrie, Vol. 115, Augsburg, September 1, 1989, NR14-1.

The final products were neutralized by addition of 85% phosphoric acid and inhibited by addition of 100 ppm t-butylhydroxytoluene.

### The Utility of the Liquid Polyoxyalkylene Copolymers of the Present Invention

The aqueous gel profiles of the liquid polyoxyalkylene block copolymers of this invention were compared with a aqueous gel prepared with a conventional solid triblock copolymer and the results are shown in Table 2. The solid, triblock copolymer used as a comparative example in this study, is poloxamer 407 available from BASF, Mt. Olive, NJ under the tradename PLURONIC® F 127 surfactant. The aqueous gels were prepared by mixing the polyethers with water and storing the solutions at about 5°C for a period of 24 hours. At the end of 24 hours, the solutions were clear and upon warming to ambient temperatures produced a sparkling clear, ringing gel. The viscosity profiles of the gels were measured using a Brookfield HBDV-III viscometer using spindles CP40 and CP51. Table 2 shows that the gel profiles of the liquid polyethers on this invention are essentially similar to those observed with Poloxamer 407. Furthermore, surprisingly the lower transition temperature for the liquid polyethers of this invention are very nearly similar to that observed with Poloxamer 407. The similarities in the lower transition temperatures allows the liquid polyethers of this invention to be used in oral care formulations in the same way as the solid, triblock copolymers. In Table 2, Sample A (CsOH catalyzed) shows a significant improvement over Sample A which was catalyzed with KOH.

**TABLE 2**

| PHYSICAL PROPERTIES OF THE PRESENT INVENTION COMPARED TO A PRIOR ART COMPOUND (POLOXAMER 407) | | |
|---|---|---|
| Sample | Pour Point/(Melt Point) °F | Form |
| A | 68 | liquid |
| A(CsOH) | 65 | liquid |
| B | 50 | liquid |
| C | 36 | liquid |
| Poloxamer 407 | 140 | solid |

Poloxamer 467 is a triblock copolymer of EO and PO with the following ideal structure as defined in the Official Monograph NF 18 for poloxamer block copolymers:

HO(C₂H₄O)₁₀₁(C₃H₆O)₅₆(C₂H₄O)₁₀₁H

Sample A of the present invention has the ideal polymer structure represented by:

HO[(C₂H₄O)₈₃/(C₃H₆O)₁₇]-[C₃H₆O]₅₆-[(C₂H₄O)₈₃/(C₃H₆O)₁₇]H

Sample B of the present invention has the polymer structure

HO[(C₂H₄O)₈₁/(C₃H₆O)₂₀]-[C₃H₆O]₅₆-[(C₂H₄O)₈₁/(C₃H₆O)₂₀]H

Sample C of the present invention has the polymer structure

HO[(C₂H₄O)₇₆/(C₃H₆O)₂₅]-[C₃H₆O]₅₆-[(C₂H₄O)₇₆/(C₃H₆O)₂₅]H

All samples are catalyzed by KOH unless otherwise indicated.

### Utility of The Liquid Polyoxyalkylene Copolymers of the Present Invention in Oral Care Compositions

Aqueous oral care compositions comprising the liquid copolymers of the present invention preferably contain 0.1 - 50% of the liquid copolymer by weight of the composition; more preferably 0.5 - 25%; most preferably 1 - 20%.

Typical aqueous based dentifrice compositions comprising the liquid copolymer of the invention may further contain other ingredients such as surfactants selected from anionic surfactants such as sodium lauryl sulfate, sodium N-lauryl sarcosinate, sodium lauryl sulfoacetate, and sodium alkyl glyceryl ether sulfonate. Additionally, the dentifrice formulation may also include abrasives such as hydrated silica, dicalciumphosphate dihydrate, calcium carbonate, sodium bicarbonate, calcium pyrophosphate and alumina. Furthermore, the dentifrice composition can also contain humectants such as, glycerin, sorbitol, propylene glycol, xylitol and liquid polyethylene glycols. Thickening agents such as sodium carboxymethyl cellulose, cellulose ethers, xanthan gum, carageenans, sodium alginate, carbopols can also be included in the dentifrice composition. Inorganic thickeners such as silica thickeners, sodium aluminum silicates and clays can also be used to provide appropriate rheology. Also other ingredients such as tartar control agents such as tetrasodium pyrophosphate, GANTREZ polymer® S-70, sodium tripolyphosphate, and zinc citrate can be included. Dentifrice compositions may also contain peroxygen compounds selected from but not limited to hydrogen peroxide and inorganic peroxides. Dentifrice actives such as triclosan, sodium monofluor limited solubility in water. A few glycol compounds can also be used in combination with or in place of alcohol, such as glycerin, sorbitol or propylene glycol. Anti-bacterial, antimicrobial, plaque-penetrating agents also constitute essential components of a mouthwash formulation. Essential oils including, but not limited to, such as clove oil, cinnamon oil, peppermint oil, spearmint oil are also be a part of the mouthwash formulation. Anti-germicidal compounds such as the quartenary ammonium compounds also find utility in mouthwash compositions. Other aesthetic ingredients such as dyes and sweetening agents can also re incorporated into the mouthwash formulation.

The solubilization properties of the liquid polyoxyalkylene compounds of this invention were compared to conventional block copolymers solubilizers such as a solid, poloxamer 407. These are shown in Table 3. Three common flavor oils typically employed in mouthwash compositions, were selected in this study. These are cinnamon oil. peppermint oil and clove oil. In each test, 0.1% aqueous solution of the flavor oil was prepared with each flavor oil composition, the prepared solutions were hazy or cloudy in the absence of the compounds of the present invention. All additives were added at a fixed concentration of 1.5%. Table 3 shows that with cinnamon oil all three liquid polyoxyalkylene compounds of this invention were able to solubilize the flavor oil to produce a clear composition and performed similar to the control with peppermint oil. Samples A & B gave hazy compositions similar to the control. However, Sample C of the invention gave a clear composition. All three liquid polyethers of this invention gave clear compositions with dove oil and performed similar to control.

**TABLE 3**

| SOLUBILIZATION PROPERTIES OF THE LIQUID POLYOXYALKYLENE COMPOUNDS OF THE PRESENT INVENTION. | | | |
|---|---|---|---|
| # | FLAVOR OIL | % ADDITIVE | APPEARANCE |
| 1 | 0.1% Cinnamon Oil | None | Hazy |
| | | 1.5% Poloxamer 407 (control) | Clear |
| | | 1.5% Sample A | Clear |
| | | 1.5% Sample B | Clear |
| | | 1.5% Sample C | Clear |
| 2 | 0.1% Peppermint Oil | None | Hazy |
| | | 1.5% Poloxamer 407 (control) | Hazy |
| | | 1.5% Sample A | Hazy |
| | | 1.5% Sample B | Hazy |
| | | 1.5% Sample C | Clear |
| 3 | 0.1% Clove Oil | None | Hazy |
| | | 1.5% Poloxamer 407 (control) | Clear |
| | | 1.5% Sample A | Clear |
| | | 1.5% Sample B | Clear |
| | | 1.5% Sample C | Clear |

The following non-limiting examples serve to illustrate the utility of the present invention. All percentages are weight percent of the total composition.

### A toothpaste formulation:

- 0.05 - 0.2%: actives selected from sodium fluoride, stannous fluoride and sodium monofluorophosphate;
- 10 - 55%: humectants, selected from glycerin, sorbitol, propylene glycol, and polyalkylene glycol;
- 0.1 - 50%: liquid polyoxyalkylene compound of the invention of the formula II

I'[(AO)ₐ-(XO)-M]ₙ Formula II

wherein I' is a initiator or a mixture of initiators having at least two carbon atoms wherein at least one carbon atom must have the substituent selected from -OH, or -NH₂;
AO is propylen oxide;
XO is (EO)_{b}(AO)_{c} wherein EO is ethylene oxide and EO and AO are distributed randomly;
M is hydrogen or alkali metal or alkaline earth metal; and
a is an integer from 15 - 35;
b is an integer from 50 - 150;
c is an integer from 7 - 90;
n is an integer from 1 - 3;
- 10-50%: water;
- 10 - 55%: abrasives, selected from calcium pyrophosphate, dicalcium phosphate and hydrated silica;
- 2 - 10%: binders, including gum karaya, tragacanth USP, sodium alginate, Irish moss and methyl cellulose;
- 2 - 8%: surfactants, including sodium lauryl sulfate, sodium N-lauryl sarcosinate, dioctyl sodium sulfosuccinate and sodium lauryl sulfoacetate;
- 0-10%: Peroxygen compounds, selected from hydrogen peroxide and inorganic peroxides.

### A mouthwash formulation:

- 0.01 - 0.1%: anti-bacterial agents, including phenolic compounds, beta-naphthol, thymol, chlorothymol and hexylresorcinol;
- 5 - 25%: humectants, including glycerol, sorbitol, propylene glycol, and polyalkylene glycol;
- 0.01 - 0.2%: essential oils, including clove oil, peppermint oil and spearmint oil;
- 0 - 30%: ethyl alcohol;
- 0.1 - 5%: liquid polyoxyalkylene compound of the invention of the formula II:

I'[(AO)ₐ-(XO)-M]ₙ Formula II

wherein I' is a initiator or a mixture of initiators having at least two carbon atoms wherein at least one carbon atom must have the substituent selected from the group -OH, or -NH₂;
AO is propylene oxide; XO is (EO)_{b}(AO)_{c} wherein EO is ethylene oxide and
EO and AO are distributed randomly;
M is hydrogen or alkali metal or alkaline earth metal; and
a is an integer from 15 - 35,
b is an integer from 50 - 150,
c is an integer from 7 - 90,
n is an integer from 1 - 3;
- 40 - 80%: Water.

## Claims

1. An oral care composition comprising 0.1-50% of the liquid polyoxyalkylene compound of the formula II:
I'[(AO)ₐ-(XO)-M]ₙ Formula II
wherein I' is an initiator or a mixture of initiators having at least two carbon atoms wherein at least one carbon atom must have the substituent selected from -OH or -NH₂; AO is propylene oxide;
XO is (EO)_{b}(AO)_{c} wherein EO is ethylene oxide and EO and AO are distributed randomly;
M is hydrogen or an alkali metal or an alkaline earth metal;
a is an integer from 15 - 35;
b is an integer from 50 - 150;
c is an integer from 7 - 90;
n is an integer from 1 - 3.

2. An oral care composition according to Claim 1, wherein a is 20 - 32, b is 65 - 135, c is 10 - 70, and n is 2 - 3.

3. An oral care composition according to Claim 1, wherein a is 24 - 31, b is 70 - 120, c is 15 - 45, and n is 2.

4. An oral care composition according to claim 1 comprising:
0.05 - 0.2% actives;
10 - 55% humectants;
0.1 - 50% liquid polyoxyalkylene compound of the formula II:
I'[(AO)ₐ-(XO)-M]ₙ Formula II
wherein I' is an initiator or a mixture of initiators having at least two carbon atoms wherein at least one carbon atom must have the substituent selected from -OH or -NH₂;
AO is propylene oxide;
XO is (EO)_{b}(AO)_{c} wherein EO is ethylene oxide and EO and AO are distributed randomly;
M is hydrogen or an alkali metal or an alkaline earth metal;
a is an integer from 15 - 35;
b is an integer from 50 - 150;
c is an integer from 7 - 90;
n is an integer from 1 - 3;
10 - 50% water;
10 - 55% abrasives;
2 - 10% binders;
2 - 8% surfactants;
0 - 10% Peroxygen compounds,
wherein the oral care composition is a toothpaste composition.

5. An oral care composition according to claim 1 comprising:
0.01 - 0.1% anti-bacterial agents;
5 - 25% humectants;
0.01 - 0.2% essential oils;
0 - 30% ethylalcohol;
0.1 - 5% the liquid polyoxyalkylene compound of the formula II:
I'[(AO)ₐ-(XO)-M]ₙ Formula II
wherein I' is an initiator or a mixture of initiators having at least two carbon atoms wherein at least one carbon atom must have the substituent selected from -OH, or -NH₂;
AO propylene oxide;
XO is (EO)_{b}(AO)_{c} wherein EO is ethylene oxide and EO and AO are distributed randomly;
M is hydrogen or alkali metal or alkaline earth metal;
a is an integer from about 15 - 35;
b is an integer from about 50 - 150;
c is an integer from about 7 - 90;
n is an integer from 1 - 3;
40 - 80%. water,
wherein the oral care composition is a mouthwash composition.

6. An oral care composition according to claim 1 comprising:
(a) a liquid polyalkylene glycol carrier and;
(b) a liquid polyoxyalkylene compound miscible in said liquid polyalkylene glycol carrier, wherein said:
liquid polyalkylene glycol carrier (a) is selected from:
(i) liquid polyethylene glycols having a molecular weight of no more than 600;
(ii)liquid polyoxyalkylene glycols having a molecular weight greater than 600 and less than 3000 of Formula I or mixtures of (i) and (ii):
I-[(EO)ₐ(AO)_{b}-M]_{y}; Formula I
wherein in Formula I:
I is an initiator or a mixture of initiators having at least two carbon atoms and at least two or more hydroxyl substituents;
AO is a C₃₋₄ alkylene oxide or a mixture of C₃₋₄ alkylene oxides;
EO is ethylene oxide;
wherein, further EO and AO can be distributed randomly and/or arranged in a block sequence;
M is hydrogen or an alkali metal or alkaline earth metal;
a is an integer from about 6 - 22;
b is an integer from about 1 - 9;
y is an integer from about 2 - 4;
further provided that
said liquid polyoxyalkylene compound (b), which is miscible in (a), is represented by Formula IIa:
I[(AO)ₐ-(XO)-M]ₓ Formula IIa
wherein in Formula IIa:
I is an initiator or a mixture of initiators having at least two carbon atoms and two hydroxyl substituents;
AO is propylene oxide;
XO is (EO)_{b}(AO)_{c} wherein EO is ethylene oxide and EO and AO are distributed randomly;
M is hydrogen or an alkali metal or an alkaline earth metal; a is an integer from 15 - 35;
b is an integer from 50 - 150;
c is an integer from 7 - 90;
x is 2.

7. A composition according to Claim 6, wherein in Formula I, a is 7 - 20; b is 1 - 7; y is 2; I is propylene glycol and M is hydrogen.

8. A composition according to Claim 6, wherein in Formula IIa, a is 20 - 32; b is 65 - 135; c is 10 - 70; x is 2 and I is propylene glycol.

9. A composition according to Claim 6, wherein in Formula I, a is 10 - 16; b is 2 - 5; and in Formula IIa, a is 24 - 31; b is 70 - 120; c is 15 - 45.

10. An oral care composition according to claim 6 comprising:
1 - 55% abrasive;
0 - 0.6% of a fluoridating agent;
2 - 10% binders;
0 - 5% thickening agent;
0 - 10% of a peroxygen source;
0 - 8% surfactants;
0.1 - 10% of the liquid polyoxyalkylene compound (b) represented by Formula IIa:
I[(AO)ₐ-(XO)-M]ₓ Formula IIa
wherein in Formula IIa:
I is an initiator or a mixture of initiators having at least two carbon atoms and two hydroxyl substituents;
AO is propylene oxide;
XO is (EO)_{b}(AO)_{c} wherein EO is ethylene oxide and EO and AO are distributed randomly;
M is hydrogen or an alkali metal or an alkaline earth metal;
a is an integer from 15 - 35;
b is an integer from 50 - 150;
c is an integer from 7 - 90;
x is 2;
5 - 70% carriers selected from the group glycerin, propylene glycol, sorbitol, liquid polyethylene glycol, and the liquid polyalkylene glycols (a) represented by Formula I:
I-[(EO)ₐ(AO)_{b}-M]_{y}; Formula I
wherein in Formula I:
I is an initiator or a mixture of initiators having at least two carbon atoms and at least two or more hydroxyl substituents;
AO is a C₃₋₄ alkylene oxide or a mixture of C₃₋₄ alkylene oxides;
EO is ethylene oxide;
wherein, further EO and AO can be distributed randomly and/or arranged in a block sequence;
M is hydrogen or an alkali metal or alkaline earth metal;
a is an integer from about 6 - 22;
b is an integer from about 1 - 9;
y is an integer from about 2 - 4,
wherein the oral care composition is a dentifrice composition.

## Patentansprüche

1. Mundpflegezusammensetzung, enthaltend 0,1-50% der flüssigen Polyoxyalkylenverbindung der Formel II:
I' [(AO)ₐ-(XO)-M]ₙ Formel II
worin I' für einen Initiator oder eine Mischung von Initiatoren mit mindestens zwei Kohlenstoffatomen, wobei mindestens ein Kohlenstoffatom einen unter -OH und -NH₂ ausgewählten Substituenten enthalten muß, steht;
AO für Propylenoxid steht;
XO für (EO)_{b}(AO)_{c}, worin EO Ethylenoxid bedeutet und EO und AO statistisch verteilt sind, steht;
M für Wasserstoff oder ein Alkali- oder Erdalkalimetall steht;
a für eine ganze Zahl von 15-35 steht;
b für eine ganze Zahl von 50-150 steht;
c für eine ganze Zahl von 7-90 steht;
n für eine ganze Zahl von 1-3 steht.

2. Mundpflegezusammensetzung nach Anspruch 1, bei der a für 20-32 steht, b für 65-135 steht, c für 10-70 steht und n für 2-3 steht.

3. Mundpflegezusammensetzung nach Anspruch 1, bei der a für 24-31 steht, b für 70-120 steht, c für 15-45 steht und n für 2 steht.

4. Mundpflegezusammensetzung nach Anspruch 1, enthaltend:
0,05-0,2% Wirkstoffe;
10-55% Feuchthaltemittel;
0,1-50% flüssige Polyoxyalkylenverbindung der Formel II:
I' [(AO)ₐ-(XO)-M]ₙ Formel II
worin I' für einen Initiator oder eine Mischung von Initiatoren mit mindestens zwei Kohlenstoffatomen, wobei mindestens ein Kohlenstoffatom einen unter -OH und -NH₂ ausgewählten Substituenten enthalten muß, steht;
AO für Propylenoxid steht;
XO für (EO)_{b}(AO)_{c}, worin EO Ethylenoxid bedeutet und EO und AO statistisch verteilt sind, steht;
M für Wasserstoff oder ein Alkali- oder Erdalkalimetall steht;
a für eine ganze Zahl von 15-35 steht;
b für eine ganze Zahl von 50-150 steht;
c für eine ganze Zahl von 7-90 steht;
n für eine ganze Zahl von 1-3 steht;
10-50% Wasser;
10-55% Abrasiva;
2-10% Bindemittel;
2-8% Tenside;
0-10% Persauerstoffverbindungen,
wobei es sich bei der Mundpflegezusammensetzung um eine Zahnpastazusammensetzung handelt.

5. Mundpflegezusammensetzung nach Anspruch 1, enthaltend:
0,01-0,1% antibakterielle Mittel;
5-25% Feuchthaltemittel;
0,01-0,2% etherische Öle;
0-30% Ethylalkohol;
0,1-5% der flüssigen Polyoxyalkylenverbindung der Formel II:
I' [(AO)ₐ-(XO)-M]ₙ Formel II
worin I' für einen Initiator oder eine Mischung von Initiatoren mit mindestens zwei Kohlenstoffatomen, wobei mindestens ein Kohlenstoffatom einen unter -OH und -NH₂ ausgewählten Substituenten enthalten muß, steht;
AO für Propylenoxid steht;
XO für (EO)_{b}(AO)_{c}, worin EO Ethylenoxid bedeutet und EO und AO statistisch verteilt sind, steht;
M für Wasserstoff oder ein Alkali- oder Erdalkalimetall steht;
a für eine ganze Zahl von 15-35 steht;
b für eine ganze Zahl von 50-150 steht;
c für eine ganze Zahl von 7-90 steht;
n für eine ganze Zahl von 1-3 steht;
40-80% Wasser;
wobei es sich bei der Mundpflegezusammensetzung um eine Mundwasserzusammensetzung handelt.

6. Mundpflegezusammensetzung nach Anspruch 1, enthaltend:
(a) einen flüssigen Polyalkylenglykolträger und
(b) eine mit dem flüssigen Polyalkylenglykolträger mischbare flüssige Polyoxyalkylenverbindung, bei der:
der flüssige Polyoxyalkylenglykolträger (a) ausgewählt ist unter:
(i) flüssigen Polyethylenglykolen mit einem Molekulargewicht von höchstens 600;
(ii) flüssigen Polyoxyalkylenglykolen mit einem Molekulargewicht von mehr als 600 und weniger als 3000 der Formel I oder Mischungen von (i) und (ii):
I-[(EO)ₐ(AO)_{b}-M]_{y} Formel I
wobei in Formel I:
I für einen Initiator oder eine Mischung von Initiatoren mit mindestens zwei Kohlenstoffatomen und mindestens zwei oder mehr Hydroxylsubstituenten steht;
AO für ein C₃₋₄-Alkylenoxid oder eine Mischung von C₃₋₄-Alkylenoxiden steht;
EO für Ethylenoxid steht;
worin ferner EO und AO statistisch verteilt und/oder in blockartiger Abfolge angeordnet sein können;
M für Wasserstoff oder ein Alkali- oder Erdalkalimetall steht;
a für eine ganze Zahl von 6-22 steht;
b für eine ganze Zahl von 1-9 steht;
y für eine ganze Zahl von 2-4 steht;
ferner mit der Maßgabe, daß
die mit (a) mischbare flüssige Polyoxyalkylenverbindung (b) durch die Formel IIa wiedergegeben wird:
I[(AO)ₐ-(XO)-M]ₓ Formel IIa
wobei in Formel IIa:
I für einen Initiator oder eine Mischung von Initiatoren mit mindestens zwei Kohlenstoffatomen und zwei Hydroxylsubstituenten steht;
AO für Propylenoxid steht;
XO für (EO)_{b}(AO)_{c}, worin EO Ethylenoxid bedeutet und EO und AO statistisch verteilt sind, steht;
M für Wasserstoff oder ein Alkali- oder Erdalkalimetall steht;
a für eine ganze Zahl von 15-35 steht;
b für eine ganze Zahl von 50-150 steht;
c für eine ganze Zahl von 7-90 steht;
x für 2 steht.

7. Zusammensetzung nach Anspruch 6, bei der in Formel I a für 7-20 steht; b für 1-7 steht; y für 2 steht; I für Propylenglykol steht und M für Wasserstoff steht.

8. Zusammensetzung nach Anspruch 6, bei der in Formel IIa a für 20-32 steht; b für 65-135 steht; c für 10-70 steht; x für 2 steht und I für Propylenglykol steht.

9. Zusammensetzung nach Anspruch 6, bei der in Formel I a für 10-16 steht und b für 2-5 steht und in Formel IIa a für 24-31 steht; b für 70-120 steht und c für 15-45 steht.

10. Mundpflegezusammensetzung nach Anspruch 6, enthaltend:
1-55% Abrasivum;
0-0,6% Fluoridierungsmittel;
2-10% Bindemittel;
0-5% Verdickungsmittel;
0-10% einer Persauerstoffquelle;
0-8% Tenside;
0,1-10% der flüssigen Polyoxyalkylenverbindung (b) der Formel IIa:
I[(AO)ₐ-(XO)-M]ₓ Formel IIa
wobei in Formel IIa:
I für einen Initiator oder eine Mischung von Initiatoren mit mindestens zwei Kohlenstoffatomen und zwei Hydroxylsubstituenten steht;
AO für Propylenoxid steht;
XO für (EO)_{b}(AO)_{c}, worin EO Ethylenoxid bedeutet und EO und AO statistisch verteilt sind, steht;
M für Wasserstoff oder ein Alkali- oder Erdalkalimetall steht;
a für eine ganze Zahl von 15-35 steht;
b für eine ganze Zahl von 50-150 steht;
c für eine ganze Zahl von 7-90 steht;
x für 2 steht;
5-70% Träger aus der Gruppe bestehend aus Glycerin, Propylenglykol, Sorbit, flüssigem Polyethylenglykol und den flüssigen Polyalkylenglykolen (a) der Formel I:
I-[(EO)ₐ(AO)_{b}-M]_{y} Formel I
wobei in Formel I:
I für einen Initiator oder eine Mischung von Initiatoren mit mindestens zwei Kohlenstoffatomen und mindestens zwei oder mehr Hydroxylsubstituenten steht;
AO für ein C₃₋₄-Alkylenoxid oder eine Mischung von C₃₋₄-Alkylenoxiden steht;
EO für Ethylenoxid steht;
worin ferner EO und AO statistisch verteilt und/oder in blockartiger Abfolge angeordnet sein können;
M für Wasserstoff oder ein Alkali- oder Erdalkalimetall steht;
a für eine ganze Zahl von etwa 6-22 steht;
b für eine ganze Zahl von etwa 1-9 steht;
y für eine ganze Zahl von etwa 2-4 steht;
wobei es sich bei der Mundpflegezusammensetzung um eine Zahnreinigungszusammensetzung handelt.

## Revendications

1. Composition d'hygiène buccale comprenant 0,1 à 50 % du composé de polyoxyalkylène liquide de la formule II :
I'[(AO)ₐ-(XO)-M]ₙ Formule II
dans laquelle I' est un amorceur ou un mélange d'amorceurs comportant au moins deux atomes de carbone, parmi lesquels au moins un atome de carbone doit présenter le substituant choisi parmi -OH ou -NH₂,
AO représente de l'oxyde de propylène,
XO représente (EO)_{b}(AO)_{c} où EO est de l'oxyde d'éthylène et EO et AO sont distribués de manière aléatoire,
M est de l'hydrogène ou un métal alcalin ou un métal alcalino-terreux,
a est un nombre entier de 15 - 35,
b est un nombre entier de 50 - 150,
c est un nombre entier de 7 - 90,
n est un nombre entier de 1 - 3.

2. Composition d'hygiène buccale suivant la revendication 1, dans laquelle a vaut 20 - 32, b vaut 65 - 135, c vaut 10 - 70 et n vaut 2 - 3.

3. Composition d'hygiène buccale suivant la revendication 1, dans laquelle a vaut 24 - 31, b vaut 70 - 120, c vaut 15 - 45 et n vaut 2.

4. Composition d'hygiène buccale suivant la revendication 1, comprenant
0,05 - 0,2 % de substances actives,
10 - 55 % d'agents humectants,
0,1 - 50 % de composé de polyoxyalkylène liquide de la formule II :
I'[(AO)ₐ-(XO)-M]ₙ Formule II
dans laquelle I' est un amorceur ou un mélange d'amorceurs comportant au moins deux atomes de carbone, parmi lesquels au moins un atome de carbone doit présenter le substituant choisi parmi -OH ou -NH₂,
AO représente de l'oxyde de propylène,
XO représente (EO)_{b}(AO)_{c} où EO est de l'oxyde d'éthylène et EO et AO sont distribués de manière aléatoire,
M est de l'hydrogène ou un métal alcalin ou un métal alcalino-terreux,
a est un nombre entier de 15 - 35,
b est un nombre entier de 50 - 150,
c est un nombre entier de 7 - 90,
n est un nombre entier de 1 - 3,
10 - 50 % d'eau,
10 - 55 % d'agents abrasifs,
2 - 10 % de liants,
2 - 8 % d'agents tensioactifs,
0 - 10 % de composés peroxygénés,
la composition d'hygiène buccale étant une composition de pâte dentifrice.

5. Composition d'hygiène buccale suivant la revendication 1, comprenant
0,01 - 0,1 % d'agents antibactériens,
5 - 25 % d'agents humectants,
0,01 - 0,2 % d'huiles essentielles,
0 - 30 % d'alcool éthylique,
0,1 - 5 % de composé de polyoxyalkylène liquide de la formule II :
I' [(AO)ₐ-(XO)-M]ₙ Formule II
dans laquelle I' est un amorceur ou un mélange d'amorceurs comportant au moins deux atomes de carbone, parmi lesquels au moins un atome de carbone doit présenter le substituant choisi parmi -OH ou -NH₂,
AO représente de l'oxyde de propylène,
XO représente (EO)_{b}(AO)_{c} où EO est de l'oxyde d'éthylène et EO et AO sont distribués de manière aléatoire,
M est de l'hydrogène ou un métal alcalin ou un métal alcalino-terreux,
a est un nombre entier d'environ 15 - 35,
b est un nombre entier d'environ 50 - 150,
c est un nombre entier d'environ 7 - 90,
n est un nombre entier de 1 - 3,
40 - 80 % d'eau,
la composition d'hygiène buccale étant une composition de bain de bouche.

6. Composition d'hygiène buccale suivant la revendication 1, comprenant
(a) un support à base de polyalkylène glycol liquide, et
(b) un composé de polyoxyalkylène liquide miscible dans ledit support à base de polyalkylène glycol liquide, dans laquelle
ledit support à base de polyalkylène glycol liquide
(a) est choisi parmi
(i) des polyéthylène glycols liquides présentant un poids moléculaire non supérieur à 600,
(ii) des polyoxyalkylène glycols liquides présentant un poids moléculaire supérieur à 600 et inférieur à 3 000 de la formule I ou des mélanges de (i) et (ii) :
I-[(EO)ₐ(AO)_{b}-M]_{y} Formule I
dans laquelle
I est un amorceur ou un mélange d'amorceurs comportant au moins deux atomes de carbone et au moins deux substituants hydroxyle ou davantage,
AO est un oxyde d'alkylène en C₃-C₄ ou un mélange d'oxydes d'alkylène en C₃-C₄,
EO représente de l'oxyde d'éthylène,
EO et AO pouvant en outre être distribués de manière aléatoire et/ou agencés dans une séquence de blocs,
M est de l'hydrogène ou un métal alcalin ou un métal alcalino-terreux,
a est un nombre entier d'environ 6 - 22,
b est un nombre entier d'environ 1 - 9,
y est un nombre entier d'environ 2 - 4,
pourvu en outre que
ledit composé de polyoxyalkylène liquide (b), qui est miscible dans (a), soit représenté par la formule IIa :
I[(AO)ₐ-(XO)-M]ₓ Formule IIa
dans laquelle
I est un amorceur ou un mélange d'amorceurs comportant au moins deux atomes de carbone et deux substituants hydroxyle,
AO représente de l'oxyde de propylène,
XO représente (EO)_{b}(AO)_{c} où EO est de l'oxyde d'éthylène et EO et AO sont distribués de manière aléatoire,
M est de l'hydrogène ou un métal alcalin ou un métal alcalino-terreux,
a est un nombre entier de 15 - 35,
b est un nombre entier de 50 - 150,
c est un nombre entier de 7 - 90,
x vaut 2.

7. Composition suivant la revendication 6, dans laquelle, dans la formule I, a vaut 7 - 20, b vaut 1 - 7, y vaut 2, I est du propylène glycol et M est de l'hydrogène.

8. Composition suivant la revendication 6, dans laquelle, dans la formule IIa, a vaut 20 - 32, b vaut 65 - 135, c vaut 10 - 70, x vaut 2 et I est du propylène glycol.

9. Composition suivant la revendication 6, dans laquelle, dans la formule I, a vaut 10 - 16, b vaut 2 - 5 et, dans la formule IIa, a vaut 24 - 31, b vaut 70 - 120 et c vaut 15 - 45.

10. Composition d'hygiène buccale suivant la revendication 6, comprenant
1 - 55 % d'agents abrasifs,
0 - 0,6 % d'un agent fluorurant,
2 - 10 % de liants,
0 - 5 % d'agent épaississant,
0 - 10 % d'une source peroxygénée,
0 - 8 % d'agents tensioactifs,
0,1 - 10 % du composé de polyoxyalkylène liquide (b) représenté par la formule IIa :
I[(AO)ₐ-(XO)-M]ₓ Formule IIa
dans laquelle
I est un amorceur ou un mélange d'amorceurs comportant au moins deux atomes de carbone et deux substituants hydroxyle,
AO représente de l'oxyde de propylène,
XO représente (EO)_{b}(AO)_{c} où EO est de l'oxyde d'éthylène et EO et AO sont distribués de manière aléatoire,
M est de l'hydrogène ou un métal alcalin ou un métal alcalino-terreux,
a est un nombre entier de 15 - 35,
b est un nombre entier de 50 - 150,
c est un nombre entier de 7 - 90,
x vaut 2.
5 - 70 % de supports choisis parmi le groupe de la glycérine, du propylène glycol, du sorbitol, du polyéthylène glycol liquide et des polyalkylène glycols liquides (a) représentés par la formule I :
I-[(EO)ₐ(AO)_{b}-M]_{y} Formule I
dans laquelle
I est un amorceur ou un mélange d'amorceurs comportant au moins deux atomes de carbone et au moins deux substituants hydroxyle ou davantage,
AO est un oxyde d'alkylène en C₃-C₄ ou un mélange d'oxydes d'alkylène en C₃-C₄,
EO représente de l'oxyde d'éthylène,
EO et AO pouvant en outre être distribués de manière aléatoire et/ou agencés dans une séquence de blocs,
M est de l'hydrogène ou un métal alcalin ou un métal alcalino-terreux,
a est un nombre entier d'environ 6 - 22,
b est un nombre entier d'environ 1 - 9,
y est un nombre entier d'environ 2 - 4,
la composition d'hygiène buccale étant une composition dentifrice.
